# EUROPEAN PATENT APPLICATION

(11) **EP 4 843 951 A1**
(43) Date of publication of application: **25.11.2026**
(21) Application number: 24925649.6
(22) Date of filing: 25.12.2024
(51) Int. Cl.: C01B 39/48, B01J 29/70, C07D 313/04

(54) **SN-BETA MOLECULAR SIEVE, PREPARATION METHOD THEREFOR AND USE THEREOF, AND OXIDATION REACTION METHOD FOR CYCLIC KETONE**

(30) Priority: 20.02.2024 CN 202410187146
(71) Applicant: China Petroleum & Chemical Corporation, Beijing 100728 (CN); Sinopec Dalian Research Institute of Petroleum and Petrochemicals Co., Ltd., Lushunkou District Dalian, Liaoning 116045 (CN)
(72) Inventor: XUE, Jinghang, Dalian, Liaoning 116045 (CN); LIU, Wei, Dalian, Liaoning 116045 (CN); QIN, Bo, Dalian, Liaoning 116045 (CN); ZHANG, Tong, Dalian, Liaoning 116045 (CN); GAO, Hang, Dalian, Liaoning 116045 (CN)
(74) Representative: karo IP
(86) International application number: PCT/CN2024/142403
(87) International publication number: WO 2025/175912

(57) **Abstract**

A Sn-Beta molecular sieve, a preparation method therefor and a use thereof, and an oxidation reaction method for a cyclic ketone. The silicon-to-tin molar ratio of the molecular sieve is 8-15, and the molecular sieve has a Lewis acid content of 600-900 µmol/g as measured by pyridine infrared spectroscopy at a desorption temperature of 200°C. The Sn-Beta molecular sieve has high Sn content and high Lewis acid content, and has excellent catalytic performance for Baeyer-Villiger oxidation reaction of cyclic ketones.

## Description

### CROSS-REFERENCE TO RELATED APPLICATIONS

The application claims the benefit of China patent application No. "202410187146.5", filed on February 20, 2024, the content of which is specifically and entirely incorporated herein by reference.

### TECHNICAL FIELD

The present invention relates to the field of molecular sieve synthesis, in particular to a Sn-Beta molecular sieve, a preparation method therefor and a use thereof, and an oxidation reaction method for a cyclic ketone.

### BACKGROUND ART

Mobil Corporation first synthesized beta molecular sieve in 1967, and the beta molecular sieve was widely applied since its synthesis. The beta zeolite exhibited excellent thermal stability, acid resistance, coking resistance and catalytic activity in a range of catalytic reactions due to its unique topological structure, high silica-alumina ratio and strong acidity. When the application values of beta zeolite in the petrochemical industry are gradually understood by the people, the research on synthesis of beta molecular sieve has deepened. Introducing heteroatoms into the beta zeolite skeleton is one of the key approaches to improving the catalytic performance of molecular sieves. Heteroatom molecular sieves are molecular sieves containing other elements, formed by replacing part of the silicon, aluminum, or phosphorus in the molecular sieve skeleton with other elements. The introduced heteroatoms may be metal atoms or non-metal atoms, after the introduction of heteroatoms, the molecular sieve can retain its original skeleton configuration.

In recent years, Sn-Beta molecular sieves have attracted the attention of numerous researchers. The molecular sieves can be used for catalyzing a variety of chemical reactions, such as catalyzing the Baeyer-Villiger oxidation reaction of cyclohexanone. The Sn atoms in skeleton of the Sn-Beta molecular sieve possess Lewis acidity, are capable of activating the carbonyl group of cyclohexanone. Subsequently, H₂O₂ inserts the oxygen atom into the position of the Sn-activated carbonyl group, and the intermediate undergoes structural rearrangement to form ε-caprolactone. Sn-Beta molecular sieve is a very promising novel molecular sieve material, but because of the mismatch between the radius of the Sn atom and the radius of the Si and Al atoms in the molecular sieve, it is quite difficult to embed Sn atoms into the skeleton of the Silica-alumina molecular sieve.

In the literature "Sn-zeolite beta as a heterogeneous chemoselective catalyst for Baeyer-Villiger oxidations" (NATURE, Vol. 412), Avelino Corma et al. reported a Sn-beta molecular sieve and its synthesis method. The literature uses tetraethyl orthosilicate (TEOS) as a silicon source, SnCl₄•5H₂O as a tin source, and HF as a mineralizer, the Sn-beta molecular sieve is obtained by high-temperature crystallization for 20 days with the aid of dealuminated nano-beta seed crystal. However, the synthesis of the molecular sieve required a use of fluorine ions as a mineralizer, the synthesized molecular sieve has large crystal grains and a low tin content.

Xiaomei Yang et al. reported a Sn-beta molecular sieve in the literature "Fluoride-free and low-concentration template agent synthesis of hierarchical Sn-Beta zeolites: efficient catalysts for conversion of glucose to alkyl lactate" (Green Chemistry, Vol. 19). The method allows for the synthesis of Sn-beta molecular sieves without the use of fluoride ions and in a low-template agent environment. However, the Sn-beta molecular sieve has a silicon-tin ratio within the range of 82-157, and a relatively small specific surface area and pore volume.

William N.P. et al. reported a Sn-beta molecular sieve and its synthesis method in the literature "Synthesis of Sn-Beta with Exclusive and High Skeleton Sn Content" (ChemCatChem, Vol. 7). The literature involved with subjecting the beta molecular sieve to an acid dealumination treatment and a dehydration treatment, then impregnating it with anhydrous SnCl₄. The impregnated beta molecular sieve was washed several times with methanol to remove the non-skeleton tin, then air-dried and calcined to obtain the Sn-beta molecular sieve. Although the molecular sieve had a high ratio of Lewis acid content to B acid content, it contained a low tin content and a low Lewis acid content.

CN112678842A discloses a tin-silicon beta molecular sieve and a method for preparing the same. The Sn-beta molecular sieve is prepared by the following steps: mixing a first silicon source, a tin source, a structure-directing agent, and a mineralizer in the presence of an aqueous solvent to obtain a mixture for primary crystallization. A second silicon source is dispersed in water, and a precipitant is then dropwise added to obtain a colloidal sol. The primary crystallization product is mixed with the colloidal sol, followed by subjecting to a secondary crystallization to obtain a product, which is then calcined to obtain the tin-silicon molecular sieve. However, the Sn-beta molecular sieve has a large particle size, a high skeleton silicon-tin ratio, and a low molecular sieve acid content.

CN104709920A discloses a tin heteroatom-containing functional molecular sieve and a synthesis method thereof. The Sn-beta molecular sieve is prepared by uniformly mixing a tin source, a silicon source, boric acid, a template agent, water, and a crystal seed according to a certain proportion and a feeding method, crystallizing at a certain temperature, and then separating, filtering, washing, and drying the crystallization product to obtain a Sn-beta molecular sieve. However, the silicon-tin ratio of the molecular sieve is within the range of 80-1,000, the molecular sieve has a relatively high silicon-tin ratio, and the skeleton has a low tin content, which is not conducive to chemical reactions catalyzed by Lewis acids.

CN104707649A discloses a tin-containing molecular sieve with a BEA topological structure and a preparation method therefor. The Sn-beta molecular sieve first treats the beta molecular sieve with acid, alkali or hydrothermal treatment to obtain a beta molecular sieve rich in Si-OH groups; a tin source, an ionic liquid and the beta molecular sieve rich in Si-OH groups are then mixed uniformly according to a certain proportion, separated after crystallization, washed and dried to obtain the Sn-beta molecular sieve. The silicon-tin ratio of the Sn-beta molecular sieve is within the range of 10-1,000, but the skeleton Sn content of the patent is low, resulting in a low Lewis acid content and poor catalytic oxidation performance for cyclohexanone.

### SUMMARY OF THE INVENTION

In view of the defects in the prior art, the present invention provides a Sn-Beta molecular sieve, a preparation method therefor and a use thereof, and an oxidation reaction method for a cyclic ketone. The Sn-Beta molecular sieve provided by the present invention has a high Sn content and a high L acid content, and has excellent catalytic performance for the oxidation reaction of cyclic ketones.

The first aspect of the present invention provides an Sn-Beta molecular sieve, the silicon-to-tin molar ratio of the molecular sieve is within the range of 8-15, and the molecular sieve has a L acid content within the range of 600-900 µmol/g as measured by pyridine infrared spectroscopy at a desorption temperature of 200°C.

Preferably, the molecular sieve has a ratio of L acid content to B acid content within the range of 70-120, more preferably within the range of 80-100 as measured by pyridine infrared spectroscopy at a desorption temperature of 200°C.

The second aspect of the present invention provides a method for preparing Sn-Beta molecular sieve, the method comprises the following steps:
(1) Subjecting a HY molecular sieve and a tin source to a dealumination and tin supplementation reaction to obtain a Sn-Al-FAU molecular sieve;
(2) Performing a crystal transformation on the Sn-Al-FAU molecular sieve in the presence of Beta zeolite seeds to obtain a Sn-Al-Beta molecular sieve;
(3) Subjecting the Sn-Al-Beta molecular sieve to a dealumination process and then subjecting to a tin supplementation treatment.

The method provided by the present invention first moderately implement the dealumination and tin supplementation on the Y molecular sieve by using a tin source, particularly a gaseous phase tin source, to allow Sn atoms to enter the molecular sieve skeleton to obtain a Sn-Al-FAU molecular sieve, then performing a crystal transformation on the Sn-Al-FAU molecular sieve to obtain a Sn-Al-Beta molecular sieve, finally, subjecting the Sn-Al-Beta molecular sieve to a secondary dealumination treatment, and then subjecting to a tin supplementation treatment using the tin source, thereby obtaining the Sn-Beta molecular sieve with a high skeleton Sn content. The Sn-beta molecular sieve synthesized with the method has the advantages of uniform Sn species distribution and high skeleton Sn content, which is manifested by a high Lewis acid content, resulting in the high reactivity and product selectivity in the cyclic ketone oxidation reactions catalyzed by L acid.

The third aspect of the present invention provides a use of the Sn-Beta molecular sieve according to the first aspect mentioned above in a cyclic ketone oxidation reaction, a saccharide isomerization reaction, a reaction for producing methyl lactate from glucose, a reaction for producing L-lactide from lactic acid, a propane dehydrogenation reaction, or a hydrogen transfer reaction between aldehyde ketones and alcohols, preferably the use in a cyclic ketone oxidation reaction, and more preferably the use in a cyclohexanone oxidation reaction.

The fourth aspect of the present invention provides an oxidation reaction method for a cyclic ketone, the method comprises: reacting a cyclic ketone with an oxidant under oxidation reaction conditions in the presence of a solvent and a catalyst; the catalyst is the Sn-Beta molecular sieve according to the first aspect.

The Sn-Beta molecular sieve provided by the present invention has better activity in the oxidation reaction of cyclic ketones, and a high selectivity and yield for the reaction product lactone. In addition, the present invention prepares the Sn-Beta molecular sieve with the method, the operation is simple, avoids the use of fluoride ions, and significantly reduces environmental pollution.

### BRIEF DESCRIPTION OF THE DRAWINGS

FIG. 1 illustrates an XRD diffractogram of the Sn-Beta molecular sieve prepared in Example 4.
FIG. 2 illustrates a SEM image at 30k magnification of the Sn-Beta molecular sieve prepared in Example 4.

### DESCRIPTION OF THE PREFERRED EMBODIMENT

The terminals and any value of the ranges disclosed herein are not limited to the precise ranges or values, such ranges or values shall be comprehended as comprising the values adjacent to the ranges or values. As for numerical ranges, the endpoint values of the various ranges, the endpoint values and the individual point values of the various ranges, and the individual point values may be combined with one another to produce one or more new numerical ranges, which should be deemed to have been specifically disclosed herein.

Unless otherwise specified in the present invention, the percentages and percentage contents are all based on mass.

The first aspect of the present invention provides an Sn-Beta molecular sieve, the silicon-to-tin molar ratio of the molecular sieve is within the range of 8-15, and the molecular sieve has a L acid content within the range of 600-900 µmol/g as measured by pyridine infrared spectroscopy at a desorption temperature of 200°C.

In the present invention, the term "Sn-Beta molecular sieve" does not specifically define the properties and structure of the product provided by the present invention, as long as the Beta molecular sieve contains Sn element, both the silicon-tin molar ratio of the molecular sieve and the Lewis acid content as measured by pyridine infrared spectroscopy at a desorption temperature of 200°C satisfy the protection scopes of the present invention.

The crystal form of the molecular sieve can be determined by X-Ray Diffraction (XRD) characterization.

According to the present invention, the silicon-to-tin molar ratio of the molecular sieve may be 8, 9, 10, 11, 12, 13, 14 or 15. Preferably, the silicon-to-tin molar ratio of the molecular sieve is within the range of 10-12.

In the present invention, the silicon-to-tin molar ratio of the molecular sieve and the SiO₂/Al₂O₃ molar ratio of the molecular sieve are measured by inductively coupled plasma atomic emission spectroscopy (ICP-AES). Before the test, the molecular sieve solid powder is pretreated and added into a mixed solution consisting of water, nitric acid and hydrofluoric acid, the molecular sieve solid powder is heated at 150°C until the sample is completely dissolved and becomes a transparent solution. After cooling, the sample is transferred to a volumetric flask, diluting to the mark with deionized water and shaking well. The standard curve method is subsequently used for quantitative analysis. Finally, the measured corresponding element content is converted into the forms of silicon-aluminum molar ratio (SiO₂/Al₂O₃) and silicon-to-tin molar ratio (SiO₂/SnO₂).

According to the present invention, preferably, the molecular sieve has a L acid content within the range of 700-800 µmol/g as measured by pyridine infrared spectroscopy at a desorption temperature of 200°C, for example 700 µmol/g, 710 µmol/g, 720 µmol/g, 730 µmol/g, 740 µmol/g, 750 µmol/g, 760 µmol/g, 770 µmol/g, 780 µmol/g, 790 µmol/g, 800 µmol/g. The molecular sieve provided by the present invention has a higher L acid content as measured by pyridine infrared spectroscopy at a desorption temperature of 200°C.

According to the present invention, preferably, the molecular sieve has a ratio of L acid content to B acid content within the range of 70-120, such as 70, 80, 90, 100, 110, or 120, more preferably within the range of 80-100 as measured by pyridine infrared spectroscopy at a desorption temperature of 200°C. A use of the molecular sieve with an appropriate ratio of L acid content to B acid content is more conducive to improving the conversion rate and selectivity of the molecular sieve during the reaction process.

In the present invention, the content of L acid (mainly produced by the skeleton Sn atoms) and B acid (mainly produced by Al atoms) of the molecular sieve is measured by pyridine-infrared spectroscopy. Specifically, the sample is prepared into a self-supporting sheet, heated to 500°C and maintained for 2h and then vacuumed. After the sample is cooled naturally to room temperature, it is adsorbed with pyridine for 30min at room temperature, then heated to 200°C and vacuumed to remove the adsorbed pyridine molecules. The pyridine infrared spectrum at the temperature is recorded. The peak at 1450cm⁻¹ in the pyridine infrared spectrum is attributed to the L acid absorption peak generated by Sn atoms, and the peak at 1545cm⁻¹ is attributed to the B acid absorption peak generated by Al atoms. After the peak area of the spectrogram is integrated, the content of infrared L acid and the content of B acid are obtained.

The quantitative calculation of L acid can be obtained based the following formula:
$L Site density \left(μmol/g\right) = \frac{{I}_{A \left({cm}^{- 1}\right)}}{E \left(cm/μmol\right)} \times \frac{a \left({cm}^{2}\right)}{m \left(g\right)} ;$ *I_{A}* denotes the integral area of the absorption peak at 1450cm⁻¹ in the spectrogram; a and m denote the area and mass of the self-supporting sheet sample respectively; E denoted the integral molar extinction coefficient, E =1.42cm/µmol.

The quantitative calculation of B acid can be obtained based the following formula:
$B Site density \left(μmol/g\right) = \frac{{I}_{A \left({cm}^{- 1}\right)}}{E \left(cm/μmol\right)} \times \frac{a \left({cm}^{2}\right)}{m \left(g\right)} ;$ *I_{A}* denotes the integral area of the absorption peak at 1545cm⁻¹ in the spectrogram; a and m denote the area and mass of the self-supporting sheet sample respectively; E denoted the integral molar extinction coefficient, E =1.95cm/µmol.

According to the present invention, preferably, the SiO₂/Al₂O₃ molar ratio of the molecular sieve is within the range of 2,700-4,000, such as 2,700, 2,800, 2,900, 3,000, 3,100, 3,200, 3,300, 3,400, 3,500, 3,600, 3,700, 3,800, 3,900 or 4,000, more preferably within the range of 3,000-3,500.

According to the present invention, preferably, the molecular sieve has a nanoscale crystallite size, further preferably, the molecular sieve has an average crystallite size within the range of 30-100 nm, more preferably within the range of 50-70 nm. The present invention provides molecular sieve with smaller grain size, which has better catalytic oxidation performance.

In the present invention, the crystallite size refers to the maximum straight-line distance between any two different points on the crystallite. For example, when the crystallite is spherical, the crystallite size refers to its diameter.

The crystallite size of the molecular sieve of the present invention can be measured by scanning electron microscopy (SEM). When the SEM measurement is adopted, 30 SEM photos of the sample at different positions are selected, and the crystallite size of the crystallite is measured and an average result is taken to obtain the average particle diameter of the crystallite.

According to the present invention, preferably, the molecular sieve has a specific surface area within the range of 530-630m²/g, more preferably within the range of 550-600m²/g.

According to the present invention, preferably, the molecular sieve has an external specific surface area within the range of 130-180m²/g, more preferably within the range of 145-165m²/g.

According to the present invention, preferably, the molecular sieve has a pore volume within the range of 0.32-0.43mL/g, more preferably within the range of 0.36-0.4mL/g.

The molecular sieve provided by the invention has a large specific surface area, external specific surface area, and large pore volume. In the invention, the specific surface area, external specific surface area, and pore volume of the molecular sieve are measured by using the N₂ adsorption-desorption method. Before measurement, the sample is first pretreated at a temperature of 300°C for 3 hours, and then adsorbed with nitrogen gas at a temperature of 77K for testing. The specific surface area of the molecular sieve is calculated by using the BET method, the pore volume is measured at the place with p/p⁰=0.98, and the external specific surface area is obtained by using the t-Plot method.

The molecular sieves having the aforementioned properties of the present invention can achieve the purpose of the present invention, and there is no special requirement on the preparation methods. According to a preferred embodiment of the present invention, the second aspect of the present invention provides a method for preparing Sn-Beta molecular sieve, the method comprises the following steps:
(1) Subjecting a HY molecular sieve and a tin source to a dealumination and tin supplementation reaction to obtain a Sn-Al-FAU molecular sieve;
(2) Performing a crystal transformation on the Sn-Al-FAU molecular sieve in the presence of Beta zeolite seeds to obtain a Sn-Al-Beta molecular sieve;
(3) Subjecting the Sn-Al-Beta molecular sieve to a dealumination process and then subjecting to a tin supplementation treatment.

In the present invention, the HY molecular sieve raw material can be any commercially available product or any HY molecular sieve prepared according to the prior art. Preferably, the HY molecular sieve in step (1) is obtained by subjecting a NaY molecular sieve to calcination following an ammonium exchange.

Preferably, the mass content of Na₂O in the HY molecular sieve in step (1) is within the range of 0.5-1.5%, more preferably within the range of 0.8-1.2%.

According to the method provided by the present invention, the content of Na₂O in said molecular sieve is measured by X-ray fluorescence (XRF).

According to the method provided by the present invention, preferably, the SiO₂/Al₂O₃ molar ratio of the HY molecular sieve in step (1) is within the range of 3.8-5, more preferably within the range of 4.2-4.6.

The specific method for dealumination and tin supplementation in step (1) of the present invention can be selected from a wide range, as long as the purpose of dealumination and tin supplementation can be achieved. Specifically, the operation mode of dealumination and tin supplementation comprises: contacting the HY molecular sieve with a dealumination and tin supplementation agent (tin source) under the dealumination and tin supplementation reaction conditions. According to a preferred embodiment of the present invention, the tin source is provided in a gaseous phase.

According to the present invention, preferably, the dealumination and tin supplementation reaction in step (1) comprises: reacting the HY molecular sieve with a gaseous phase tin source under the dealumination and tin supplementation reaction conditions. The preferred embodiment not only facilitates the preparation of a product with further improved performance, but also facilitates the better separation of the molecular sieve and the tin source reactant after completion of the reaction.

According to the present invention, preferably, the dealumination and tin supplementation reaction conditions comprises: a reaction temperature within the range of 400-600°C, more preferably within the range of 450-550°C, a time of 0.5-5 hours, more preferably 2-3 hours; a partial pressure of the gaseous phase tin source is within the range of 2.5kPa-7.5kPa, more preferably within the range of 4kPa-6kPa. According to the present invention, preferably, the tin source is an anhydrous tin source, more preferably at least one selected from the group consisting of tin tetrachloride, methyltin-trichloride, dimethyltin-dichloride and trimethyltin-chloride.

According to the present invention, preferably, the solvent (e.g., water) is not present during the dealumination and tin supplementation reaction process.

According to a specific embodiment of the present invention, the dealumination and tin supplementation reaction is carried out under an inert gas flow state. Preferably, the inert gas flow carries the gaseous tin source into the reactor to carry out reaction with the HY molecular sieve. The inert gas is a conventional inert gas in the art, preferably, the inert gas is one selected from the group consisting of nitrogen gas, helium, argon, or a mixed gas thereof in any proportion.

According to the present invention, the dealumination and tin supplementation reaction in step (1) can be carried out under a normal pressure environment. There are no particular limitations on the specific operation mode, the conventional selection and appropriate adjustments can be implemented by those skilled in the art based on the above disclosure. For example, during the dealumination and tin supplementation reaction process, the pressure of the mixed gas inlet is slightly higher than the pressure at the outlet to maintain the forward flow of the mixed gas at a certain flow rate.

According to the present invention, preferably, the flow rate of the mixed gas of the tin source and the inert gas in the dealumination and tin supplementation reaction conditions in step (1) is within the range of 5-20 mL/(min·g).

According to the present invention, there is no particular limitation on the specific mode of carrying out the dealumination and tin supplementation reaction in step (1), which can be performed in a fixed bed reactor.

According to the present invention, preferably, the method further comprises: dehydrating the HY molecular sieve before the dealumination and tin supplementation reaction. The arrangement is more conducive to ensuring that step (1) is carried out under anhydrous conditions. Preferably, the specific method of dehydrating the HY molecular sieve comprises: dehydrating the molecular sieve under an inert atmosphere.

Preferably, the dehydration conditions comprise: a dehydration temperature within the range of 350-550°C, more preferably within the range of 400-470°C, and a dehydration time of 2-10 hours, more preferably 4-7 hours.

Preferably, the method further comprises: after the dealumination and tin supplementation reaction in step (1), stopping the introduction of the tin source, introducing an inert gas for purging, and then cooling to decrease temperature, preferably cooling to room temperature and then performing step (2).

According to a preferred embodiment of the present invention, step (2) comprises: mixing Beta zeolite seeds, a template agent, a silicon source, a Sn-Al-FAU molecular sieve and a solvent to obtain a mixed solution, then performing a crystallization reaction, and calcining the crystallized solid product.

Preferably, the molar ratio of the materials in the mixed solution is SiO₂ : (0.03-0.09) Al₂O₃ : (0.03-0.08) SnO₂ : (0.006-0.021) Na₂O : (0.1-0.8) template agent : (3-8) H₂O, more preferably SiO₂ : (0.05-0.07) Al₂O₃ : (0.05-0.07) SnO₂ : (0.009-0.018) Na₂O : (0.4-0.6) template agent : (5-7) H₂O.

Preferably, the Beta zeolite seeds calculated in terms of silica account for 3-10wt%, more preferably 6-8wt% of the total amount of silica in the mixed solution.

The present invention has wide selection ranges for the types of the Beta zeolite seeds, template agent, silicon source and solvent.

The Beta zeolite seeds may be prepared by any method in the field. Preferably, the SiO₂/Al₂O₃ molar ratio of the Beta zeolite seeds is within the range of 20-35, further preferably within the range of 25-30.

The template agent can be any template agent capable of performing crystal transformation of Sn-Al-FAU molecular sieve into Sn-Al-Beta molecular sieve. Preferably, the template is tetraethyl ammonium hydroxide. Specifically, the template agent can be provided in the form of an aqueous solution. Preferably, the concentration of the template agent aqueous solution is within the range 20-40wt%.

The silicon source can be any silicon source commonly used in the synthesis of molecular sieves in the field. Preferably, the silicon source is at least one selected from the group consisting of precipitated silica, fumed silica, and silica sol.

The solvent includes but is not limited to water.

According to a preferred embodiment of the present invention, the crystallization conditions in step (2) comprise: a crystallization temperature within the range of 138-145°C, more preferably within the range of 140-142°C; and a crystallization time of 5-15 days, more preferably 8-12 days. The crystallization can be carried out in a reaction kettle.

According to a preferred embodiment of the present invention, the crystallization is carried out under hermetic conditions.

According to a preferred embodiment of the present invention, the calcination conditions in step (2) comprise: a calcination temperature within the range of 500-600°C and a calcination time of 4-8 hours.

Preferably, the method further comprises: filtering, washing, and drying the crystallized product before the calcination in step (2). The invention does not particularly limit the filtering, washing, and drying conditions, those skilled in the field can select the conditions according to conventional technical means.

According to the present invention, preferably, the dealumination in step (3) comprises: subjecting the Sn-Al-Beta molecular sieve to an acid treatment. There is no particular limitation on the specific conditions and operation mode of the acid treatment.

According to the present invention, preferably, the dealumination in step (3) comprises: reacting the Sn-Al-Beta molecular sieve with an acid solution.

The acid used in the dealumination step (3) of the present invention has a wide selection range, it can be an inorganic acid and/or an organic acid, preferably an inorganic acid. The inorganic acid is preferably at least one selected from the group consisting of nitric acid, hydrochloric acid and sulfuric acid. The organic acid is preferably at least one selected from the group consisting of citric acid, oxalic acid, EDTA, and tartaric acid.

According to the present invention, preferably, the concentration of said acid solution is within the range of 2.5-5mol/L, more preferably within the range of 3.5-4.5mol/L.

According to the present invention, preferably, the solid-liquid mass ratio of the Sn-Al-Beta molecular sieve to the acid solution is 1:30-1:100, more preferably 1:50-1:70.

According to the present invention, preferably, the conditions for reacting the Sn-Al-Beta molecular sieve with the acid solution comprise: a temperature within the range of 30-90°C, more preferably within the range of 50-70°C; a time of 1-6 hours, more preferably 3-4 hours. The present invention does not particularly limit the number of acid treatments, which can be once, or 2 or more times, preferably, the acid treatment is performed for 2-5 times, more preferably 3-4 times.

Preferably, the method further comprises: washing, filtering, and drying the solid product obtained after the dealumination in step (3). The invention does not particularly restrain the conditions for washing, filtering, and drying, and those skilled in the field can select the conditions according to conventional technical means. The drying conditions preferably comprise a temperature of 120-160°C and a drying time of 4-6 hours.

According to the present invention, preferably, the tin supplementation treatment in step (3) comprises: reacting the dealuminated molecular sieve with a gaseous phase tin source under the tin supplementation treatment conditions. In the present invention, the specific operation mode of the tin supplementation in step (3) can be the same as that in step (1), and the content will not be repeatedly described herein.

According to a preferred embodiment of the present invention, the tin source in step (3) is provided in a gaseous phase form. According to a specific embodiment of the invention, the tin supplementation reaction is carried out under the flow state of an inert gas. The tin source is carried into the reactor by the inert gas flow to contact and react with the dealuminated molecular sieve.

According to the present invention, preferably, the tin supplementation treatment conditions comprise: a reaction temperature within the range of 400-550°C, more preferably within the range of 450-500°C, a time of 2-7 hours, more preferably 4-6 hours; a partial pressure of the gaseous phase tin source is within the range of 1kPa-4kPa, more preferably within the range of 2kPa-3kPa.

According to the present invention, specifically, the tin supplementation treatment stage is carried out under normal pressure environment, and the pressure of the gas inlet is slightly higher than the outlet pressure to maintain the forward flow of the mixed gas at a certain flow rate.

According to the present invention, preferably, the flow rate of the mixed gas of the tin source and the inert gas in the tin supplementation treatment reaction conditions in step (3) is within the range of 5-20 mL/(min·g).

The type of tin source used in the tin supplementation process can be the same as the selection range of the above step (1), the content will not be repeatedly described herein.

According to the present invention, preferably, the method further comprises: dehydrating the dealuminated Sn-Beta molecular sieve before the tin supplementation treatment. The arrangement is more conducive to ensuring that step (3) is performed under anhydrous conditions. Preferably, the specific mode of dehydrating the dealuminated Sn-Beta molecular sieve comprises: dehydrating the dealuminated Sn-Beta molecular sieve under an inert atmosphere.

The Sn-Beta molecular sieve according to the first aspect of the present invention has a wide range of application fields, it is applicable to various technical fields where the existing Sn-Beta molecular sieves can be applied, including but not limited to: a cyclic ketone oxidation reaction, a saccharide isomerization reaction, a reaction for producing methyl lactate from glucose, a reaction for producing L-lactide from lactic acid, a propane dehydrogenation reaction, or a hydrogen transfer reaction between aldehyde ketones and alcohols.

The third aspect of the present invention provides a use of the Sn-Beta molecular sieve according to the first aspect mentioned above in a cyclic ketone oxidation reaction, a saccharide isomerization reaction, a reaction for producing methyl lactate from glucose, a reaction for producing L-lactide from lactic acid, a propane dehydrogenation reaction, or a hydrogen transfer reaction between aldehyde ketones and alcohols, preferably the use in a cyclic ketone oxidation reaction. The inventors of the present invention have discovered that the Sn-Beta molecular sieve is particularly suitable for cyclic ketone oxidation reaction. The Sn-Beta molecular sieve provided by the present invention has high performance in the oxidation reaction of cyclic ketones, especially the Baeyer-Villiger oxidation reaction. The Sn-Beta molecular sieve of the present invention is suitable for the oxidation reaction of a variety of cyclic ketones, such as at least one selected from the group consisting of cyclohexanone, 2-adamantanone, dihydrocarvone and bicyclo[3.2.0]hept-2-en-6-one, preferably the use of the Sn-Beta molecular sieve in the oxidation reaction of cyclohexanone.

The fourth aspect of the present invention provides an oxidation reaction method for a cyclic ketone, the method comprises: reacting a cyclic ketone with an oxidant under oxidation reaction conditions in the presence of a solvent and a catalyst; the catalyst is the Sn-Beta molecular sieve according to the first aspect.

Preferably, the cyclic ketone is at least one selected from the group consisting of cyclohexanone, 2-adamantanone, dihydrocarvone and bicyclo[3.2.0]hept-2-en-6-one, more preferably cyclohexanone.

The present invention has a wide range of choices for the oxidant, and any conventional choice in the field can be used as long as it can oxidize the cyclic ketone. Preferably, the oxidant is at least one selected from the group consisting of tert-butyl hydroperoxide, hydrogen peroxide, cumyl hydroperoxide, cyclohexyl hydroperoxide, peracetic acid and peroxypropionic acid, more preferably hydrogen peroxide.

The present invention has a wide selection range for the type of solvent, as long as it can provide a reaction environment. Preferably, the solvent is at least one selected from the group consisting of 1,4-dioxane, decane, chlorobenzene and benzyl alcohol.

Preferably, the used amount of the cyclic ketone is 0.4-1.4 mmol relative to 1 mmol of the oxidant.

Preferably, the used amount of the catalyst is 10-25 mg relative to 1 mmol of the oxidant.

Preferably, the oxidation reaction conditions comprise: an oxidation reaction temperature within the range of 75-95°C, and an oxidation reaction time of 2-8 hours.

Unless otherwise specified in the present invention, the percentages and percentage contents are all based on mass.

The preparation process and product performance of the method of the present invention are further illustrated below with reference to examples and comparative examples, but the following examples do not impose limitations on the method of the present invention.

In molecular sieves of the following examples and comparative examples, the silicon-to-tin molar ratio and SiO₂/Al₂O₃ molar ratio of the molecular sieve were measured by inductively coupled plasma atomic emission spectroscopy (ICP-AES). The content of L acid and the content of B acid of the molecular sieves were measured by pyridine-infrared spectroscopy. The specific surface area, external specific surface area, and pore volume of the molecular sieve were measured by N₂ adsorption-desorption method.

The average crystallite size of the molecular sieve can be measured by scanning electron microscopy (SEM).

The Na₂O content in the molecular sieve was measured by X-Ray Fluorescence (XRF).

The specific conditions of each test method were as described in the DESCRIPTION OF THE PREFERRED EMBODIMENT mentioned above, the conditions were not repeatedly described herein.

The silica-alumina ratio described in the following Examples and Comparative Examples indicated the SiO₂/Al₂O₃ molar ratio.

### Example 1

A HY molecular sieve with a silica-alumina ratio of 4.0 and a Na₂O content of 0.6% was loaded into a fixed bed reactor, a nitrogen gas flow was introduced, the temperature was raised to 350°C for dehydration for 3 hours, SnCl₄ steam with a partial pressure of 7.3 kPa carried by the nitrogen gas flow entered the reactor, the temperature was further raised to 400°C. The flow rate of the mixed gas was controlled to be 12 mL/(min·g), and the reaction was carried out at the temperature for 5 hours; after the reaction, the introduction of SnCl₄ was stopped, the introduction of nitrogen gas for purging air was continued at the reaction temperature for 1 hour, the temperature was subsequently cooled to 30°C under the condition of purging with nitrogen gas, the product was taken out to obtain a Sn-Al-FAU molecular sieve.

A tetraethylammonium hydroxide (TEAOH) solution with a concentration of 40wt% was added into a certain amount of water and stirred uniformly, precipitated silica was then added and stirred uniformly, beta zeolite seeds (with a silica/alumina ratio of 20) were subsequently added, Sn-Al-FAU molecular sieve was finally added, the materials were stirring uniformly, the solution had a material molar ratio of SiO₂: 0.09Al₂O₃: 0.08SnO₂: 0.007Na₂O: 0.8TEAOH: 8H₂O, with the Beta zeolite seeds calculated in terms of silica accounting for 10wt% of the total amount of silica in the mixed solution. The materials were placed in a reaction kettle and subjected to crystallization under sealed conditions at 144°C for 6 days, the crystallization product was taken out, filtered, washed, dried, and calcined at 550°C for 6 hours, a Sn-Al-Beta molecular sieve product was obtained.

The obtained Sn-Al-Beta molecular sieve was placed in a nitric acid solution with a concentration of 2.5 mol/L for dealumination, the solid-liquid mass ratio was controlled at 1:30, the treatment was implemented at a temperature of 30°C for 1 hour, and the treatment was performed for 2 times.

The dealuminated Sn-Beta molecular sieve was loaded into a fixed bed reactor, nitrogen gas was introduced, the temperature was raised to 300°C for dehydration for 8 hours, anhydrous SnCl₄ was heated, the nitrogen flow was then used for carrying SnCl₄ saturated steam with a partial pressure of 4.0kPa into the reaction tube, the temperature was further raised to 550°C for performing a reaction for 7 hours, the flow rate of the mixed gas was controlled to be 12 mL/(min·g). After the reaction, the introduction of SnCl₄ was stopped, the introduction of nitrogen gas for purging air was continued at the reaction temperature for 1 hour, the temperature was subsequently cooled to 30°C under the nitrogen gas purging, the product was taken out, the Sn-Beta molecular sieve with a secondary tin supplementation was prepared.

### Example 2

A HY molecular sieve with a silica-alumina ratio of 5.0 and a Na₂O content of 1.4% was loaded into a fixed bed reactor, a nitrogen gas flow was introduced, the temperature was raised to 520°C for dehydration for 5 hours, SnCl₄ steam with a partial pressure of 2.6 kPa carried by the nitrogen gas flow entered the reactor, the temperature was controlled to 400°C. The flow rate of the mixed gas was controlled to be 12 mL/(min·g), and the reaction was carried out at the temperature for 0.5 hour; after the reaction, the introduction of SnCl₄ was stopped, the introduction of nitrogen gas for purging air was continued at the reaction temperature for 1 hour, the temperature was subsequently cooled to 30°C under the condition of purging with nitrogen gas, the product was taken out to obtain a Sn-Al-FAU molecular sieve.

A tetraethylammonium hydroxide (TEAOH) solution with a concentration of 40wt% was added into a certain amount of water and stirred uniformly, precipitated silica was then added and stirred uniformly, beta zeolite seeds (with a silica/alumina ratio of 34) were subsequently added, Sn-Al-FAU molecular sieve was finally added, the materials were stirring uniformly, the solution had a material molar ratio of SiO₂: 0.035Al₂O₃: 0.03SnO₂: 0.020Na₂O: 0.2TEAOH: 3H₂O, with the Beta zeolite seeds calculated in terms of silica accounting for 3wt% of the total amount of silica in the mixed solution. The materials were placed in a reaction kettle and subjected to crystallization under sealed conditions at 138°C for 15 days, the crystallization product was taken out, filtered, washed, dried, and calcined at 550°C for 6 hours, a Sn-Al-Beta molecular sieve product was obtained.

The obtained Sn-Al-Beta molecular sieve was placed in a nitric acid solution with a concentration of 5 mol/L for dealumination, the solid-liquid mass ratio was controlled at 1:100, the treatment was implemented at a temperature of 80°C for 2 hours, and the treatment was performed for 5 times.

The dealuminated Sn-Beta molecular sieve was loaded into a fixed bed reactor, nitrogen gas was introduced, the temperature was raised to 500°C for dehydration for 3 hours, anhydrous SnCl₄ was heated, the nitrogen flow was then used for carrying SnCl₄ saturated steam with a partial pressure of 1.2kPa into the reaction tube, the temperature was further raised to 420°C for performing a reaction for 2 hours, the flow rate of the mixed gas was controlled to be 12 mL/(min·g). After the reaction, the introduction of SnCl₄ was stopped, the introduction of nitrogen gas for purging air was continued at the reaction temperature for 1 hour, the temperature was subsequently cooled to 30°C under the nitrogen gas purging, the product was taken out, the Sn-Beta molecular sieve with a secondary tin supplementation was prepared.

### Example 3

A HY molecular sieve with a silica-alumina ratio of 4.1 and a Na₂O content of 1.3% was loaded into a fixed bed reactor, a nitrogen gas flow was introduced, the temperature was raised to 380°C for dehydration for 3 hours, SnCl₄ steam with a partial pressure of 6.4 kPa carried by the nitrogen gas flow entered the reactor, the temperature was further raised to 440°C. The flow rate of the mixed gas was controlled to be 12 mL/(min·g), and the reaction was carried out at the temperature for 4 hours; after the reaction, the introduction of SnCl₄ was stopped, the introduction of nitrogen gas for purging air was continued at the reaction temperature for 1 hour, the temperature was subsequently cooled to 30°C under the condition of purging with nitrogen gas, the product was taken out to obtain a Sn-Al-FAU molecular sieve.

A tetraethylammonium hydroxide (TEAOH) solution with a concentration of 40wt% was added into a certain amount of water and stirred uniformly, precipitated silica was then added and stirred uniformly, beta zeolite seeds (with a silica/alumina ratio of 32) were subsequently added, Sn-Al-FAU molecular sieve was finally added, the materials were stirring uniformly, the solution had a material molar ratio of SiO₂: 0.085Al₂O₃: 0.045SnO₂: 0.019Na₂O: 0.3TEAOH: 4H₂O, with the Beta zeolite seeds calculated in terms of silica accounting for 9wt% of the total amount of silica in the mixed solution. The materials were placed in a reaction kettle and subjected to crystallization under sealed conditions at 139°C for 7 days, the crystallization product was taken out, filtered, washed, dried, and calcined at 550°C for 6 hours, a Sn-Al-Beta molecular sieve product was obtained.

The obtained Sn-Al-Beta molecular sieve was placed in a nitric acid solution with a concentration of 3 mol/L for dealumination, the solid-liquid mass ratio was controlled at 1:80, the treatment was implemented at a temperature of 45°C for 6 hours, and the treatment was performed for 2 times.

The dealuminated Sn-Beta molecular sieve was loaded into a fixed bed reactor, nitrogen gas was introduced, the temperature was raised to 450°C for dehydration for 7 hours, anhydrous SnCl₄ was heated, the nitrogen flow was then used for carrying SnCl₄ saturated steam with a partial pressure of 3.5kPa into the reaction tube, the temperature was raised to 430°C for performing a reaction for 7 hours, the flow rate of the mixed gas was controlled to be 12 mL/(min·g). After the reaction, the introduction of SnCl₄ was stopped, the introduction of nitrogen gas for purging air was continued at the reaction temperature for 1 hour, the temperature was subsequently cooled to 30°C under the nitrogen gas purging, the product was taken out, the Sn-Beta molecular sieve with a secondary tin supplementation was prepared.

### Example 4

A HY molecular sieve with a silica-alumina ratio of 4.5 and a Na₂O content of 1.0% was loaded into a fixed bed reactor, a nitrogen gas flow was introduced, the temperature was raised to 450°C for dehydration for 6 hours, SnCl₄ steam with a partial pressure of 5.0 kPa carried by the nitrogen gas flow entered the reactor, the temperature was further raised to 500°C. The flow rate of the mixed gas was controlled to be 12 mL/(min·g), and the reaction was carried out at the temperature for 2 hours; after the reaction, the introduction of SnCl₄ was stopped, the introduction of nitrogen gas for purging air was continued at the reaction temperature for 1 hour, the temperature was subsequently cooled to 30°C under the condition of purging with nitrogen gas, the product was taken out to obtain a Sn-Al-FAU molecular sieve.

A tetraethylammonium hydroxide (TEAOH) solution with a concentration of 40wt% was added into a certain amount of water and stirred uniformly, precipitated silica was then added and stirred uniformly, beta zeolite seeds (with a silica/alumina ratio of 28) were subsequently added, Sn-Al-FAU molecular sieve was finally added, the materials were stirring uniformly, the solution had a material molar ratio of SiO₂: 0.06Al₂O₃: 0.05SnO₂: 0.014Na₂O: 0.5TEAOH: 5H₂O, with the Beta zeolite seeds calculated in terms of silica accounting for 8wt% of the total amount of silica in the mixed solution. The materials were placed in a reaction kettle and subjected to crystallization under sealed conditions at 140°C for 10 days, the crystallization product was taken out, filtered, washed, dried, and calcined at 550°C for 6 hours, a Sn-Al-Beta molecular sieve product was obtained.

The obtained Sn-Al-Beta molecular sieve was placed in a nitric acid solution with a concentration of 3.5 mol/L for dealumination, the solid-liquid mass ratio was controlled at 1:70, the treatment was implemented at a temperature of 60°C for 4 hours, and the treatment was performed for 3 times.

The dealuminated Sn-Beta molecular sieve was loaded into a fixed bed reactor, nitrogen gas was introduced, the temperature was raised to 400°C for dehydration for 5 hours, anhydrous SnCl₄ was heated, the nitrogen flow was then used for carrying SnCl₄ saturated steam with a partial pressure of 3.0kPa into the reaction tube, the temperature was further raised to 480°C for performing a reaction for 5 hours, the flow rate of the mixed gas was controlled to be 12 mL/(min·g). After the reaction, the introduction of SnCl₄ was stopped, the introduction of nitrogen gas for purging air was continued at the reaction temperature for 1 hour, the temperature was subsequently cooled to 30°C under the nitrogen gas purging, the product was taken out, the Sn-Beta molecular sieve with a secondary tin supplementation was prepared.

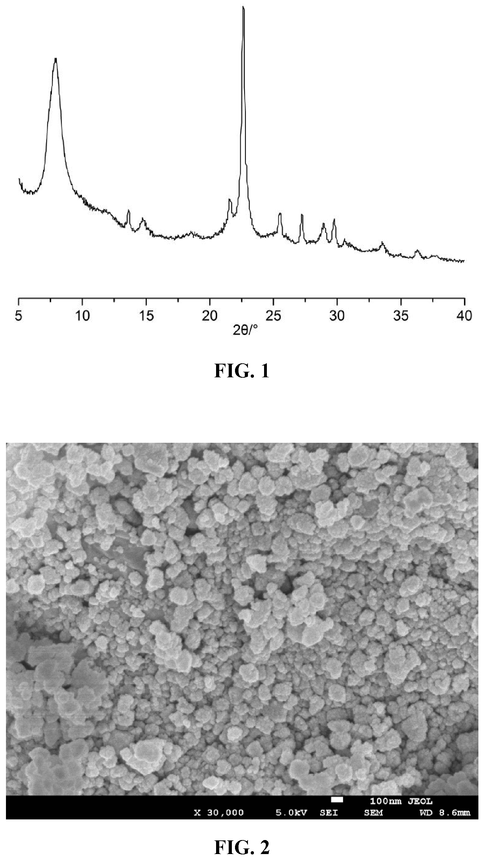

FIG. 1 illustrated an XRD diffractogram of the Sn-Beta molecular sieve prepared in Example 4. As can be seen the figure, the molecular sieve exhibited the characteristic diffraction peaks of Beta molecular sieve, and the SnO₂ diffraction peak was not present, indicated that most of Sn entered into the molecular sieve skeleton.

FIG. 2 illustrated a SEM image at 50k magnification of the Sn-Beta molecular sieve prepared in Example 4. As can be seen the figure, the molecular sieve had a nanoscale crystallite size.

### Example 5

A HY molecular sieve with a silica-alumina ratio of 4.4 and a Na₂O content of 0.8% was loaded into a fixed bed reactor, a nitrogen gas flow was introduced, the temperature was raised to 410°C for dehydration for 4 hours, SnCl₄ steam with a partial pressure of 6.0 kPa carried by the nitrogen gas flow entered the reactor, the temperature was further raised to 550°C. The flow rate of the mixed gas was controlled to be 12 mL/(min·g), and the reaction was carried out at the temperature for 3 hours; after the reaction, the introduction of SnCl₄ was stopped, the introduction of nitrogen gas for purging air was continued at the reaction temperature for 1 hour, the temperature was subsequently cooled to 30°C under the condition of purging with nitrogen gas, the product was taken out to obtain a Sn-Al-FAU molecular sieve.

A tetraethylammonium hydroxide (TEAOH) solution with a concentration of 40wt% was added into a certain amount of water and stirred uniformly, precipitated silica was then added and stirred uniformly, beta zeolite seeds (with a silica/alumina ratio of 26) were subsequently added, Sn-Al-FAU molecular sieve was finally added, the materials were stirring uniformly, the solution had a material molar ratio of SiO₂: 0.06Al₂O₃: 0.06SnO₂: 0.010Na₂O: 0.6TEAOH: 7H₂O, with the Beta zeolite seeds calculated in terms of silica accounting for 6wt% of the total amount of silica in the mixed solution. The materials were placed in a reaction kettle and subjected to crystallization under sealed conditions at 140°C for 10 days, the crystallization product was taken out, filtered, washed, dried, and calcined at 550°C for 6 hours, a Sn-Al-Beta molecular sieve product was obtained.

The obtained Sn-Al-Beta molecular sieve was placed in a nitric acid solution with a concentration of 4.5 mol/L for dealumination, the solid-liquid mass ratio was controlled at 1:50, the treatment was implemented at a temperature of 50°C for 3 hours, and the treatment was performed for 4 times.

The dealuminated Sn-Beta molecular sieve was loaded into a fixed bed reactor, nitrogen gas was introduced, the temperature was raised to 350°C for dehydration for 5 hours, anhydrous SnCl₄ was heated, the nitrogen flow was then used for carrying SnCl₄ saturated steam with a partial pressure of 2.4kPa into the reaction tube, the temperature was further raised to 460°C for performing a reaction for 5 hours, the flow rate of the mixed gas was controlled to be 12 mL/(min·g). After the reaction, the introduction of SnCl₄ was stopped, the introduction of nitrogen gas for purging air was continued at the reaction temperature for 1 hour, the temperature was subsequently cooled to 30°C under the nitrogen gas purging, the product was taken out, the Sn-Beta molecular sieve with a secondary tin supplementation was prepared.

### Comparative Example 1

A tetraethylammonium hydroxide (TEAOH) solution with a concentration of 40wt% was added into a certain amount of water and stirred uniformly, precipitated silica was then added and stirred uniformly, beta zeolite seeds (with a silica/alumina ratio of 20) were subsequently added, the HY molecular sieve with a silica-alumina ratio of 4.0 and Na₂O content of 0.6% was further added, and a certain amount of SnCl₄•5H₂O was finally added, the materials were stirring uniformly, the solution had a material molar ratio of SiO₂: 0.09Al₂O₃: 0.08SnO₂: 0.007Na₂O: 0.8TEAOH: 8H₂O, with the Beta zeolite seeds calculated in terms of silica accounting for 10wt% of the total amount of silica in the mixed solution. The materials were placed in a reaction kettle and subjected to crystallization under sealed conditions at 144°C for 6 days, the crystallization product was taken out, filtered, washed, dried, and calcined at 550°C for 6 hours, a Sn-Al-Beta molecular sieve product was obtained.

The obtained Sn-Al-Beta molecular sieve was placed in a nitric acid solution with a concentration of 2.5 mol/L for dealumination, the solid-liquid mass ratio was controlled at 1:30, the treatment was implemented at a temperature of 30°C for 1 hour, and the treatment was performed for 2 times.

The dealuminated Sn-Beta molecular sieve was loaded into a fixed bed reactor, nitrogen gas was introduced, the temperature was raised to 300°C for dehydration for 8 hours, anhydrous SnCl₄ was heated, the nitrogen flow was then used for carrying SnCl₄ saturated steam with a partial pressure of 4.0kPa into the reaction tube, the temperature was further raised to 550°C for performing a reaction for 7 hours, the flow rate of the mixed gas was controlled to be 12 mL/(min·g). After the reaction, the introduction of SnCl₄ was stopped, the introduction of nitrogen gas for purging air was continued at the reaction temperature for 1 hour, the temperature was subsequently cooled to 30°C under the nitrogen gas purging, the product was taken out, the Sn-Beta molecular sieve with a secondary tin supplementation was prepared.

### Comparative Example 2

3g of Beta molecular sieve (with a silica/alumina ratio of 21) was treated with concentrated nitric acid (with a concentration of 65%) according to the liquid:solid ratio of 20 (weight ratio) at a temperature of 100°C for 24 hours, a Beta molecular sieve with a high content of Si-OH ions (with a silica/alumina ratio larger than 2,000) was obtained.

100g of dissolved l-butyl-3-methylchloroimidazole salt ionic liquid was weighed and added into a reactor, 1.5g of tin tetrachloride was added, the materials were vigorously stirred for carrying out a reaction for 4 hours, the previously obtained Beta molecular sieve with a high content of Si-OH ions was added, the materials were vigorously stirred for carrying out a reaction for 4 hours, a uniformly mixed crystallization mixture was obtained, with a molar ratio of 1.0SiO₂:0.1SnO₂.

The obtained uniformly mixed crystallization mixture was then transferred to an open-ended reaction kettle for crystallization at a temperature of 200°C for 20 hours. After the crystallization was completed, the reactant was cooled to room temperature, filtered, washed and dried to obtain the tin-containing Beta heteroatom molecular sieve.

The structure and properties of the molecular sieves prepared in the Examples and Comparative Examples were listed in Table 1 and Table 2 below.

**Table 1**

| | Specific surface area m²/g | External specific surface area m²/g | Pore volume mL/g | Average crystallite size nm | L acid content µmol/g | Ratio of L acid content to B acid content |
|---|---|---|---|---|---|---|
| Example 1 | 536 | 130 | 0.32 | 93 | 890 | 120 |
| Example 2 | 628 | 175 | 0.43 | 34 | 630 | 70 |
| Example 3 | 602 | 142 | 0.41 | 81 | 675 | 79 |
| Example 4 | 580 | 164 | 0.40 | 55 | 756 | 84 |
| Example 5 | 557 | 152 | 0.37 | 62 | 790 | 96 |
| Comparative Example 1 | 364 | 120 | 0.22 | 156 | 307 | 53 |
| Comparative Example 2 | 550 | 140 | 0.40 | 90 | 356 | 45 |

**Table 2**

| | SiO₂/SnO₂ molar ratio | SiO₂/Al₂O₃ molar ratio | SnO₂/Al₂O₃ molar ratio |
|---|---|---|---|
| Example 1 | 8 | 3,960 | 495 |
| Example 2 | 15 | 2,750 | 183 |
| Example 3 | 14 | 3,500 | 250 |
| Example 4 | 12 | 3,120 | 260 |
| Example 5 | 10 | 3,460 | 346 |
| Comparative Example 1 | 32 | 3,300 | 103 |
| Comparative Example 2 | 13 | 2,800 | 215 |

### Test Example

The test example served to illustrate the properties of the molecular sieve provided by the present invention in the Baeyer-Villiger oxidation reaction.

The performance of the Sn-Beta molecular sieves in the Examples and Comparative Examples was evaluated by using the Baeyer-Villiger oxidation reaction of cyclohexanone and hydrogen peroxide (35 wt%). The reaction was performed in a round-bottom flask equipped with a condensate water cooling unit. 50 mg of the catalyst was weighed and added into the flask, 10mL of 1,4-dioxane (solvent), 2 mmol of cyclohexanone, 3 mmol of H₂O₂ (35wt%), and 0.23g of chlorobenzene (internal standard) were subsequently added, the reaction solution was placed in an oil bath pan with a temperature of 90°C for carrying out a reaction for 3 hours; after completion of the reaction, the reaction solution was transferred to ice water and cooled to room temperature, and subjected to centrifugation, the supernatant was analyzed by gas chromatography for measuring the cyclohexanone conversion rate, selectivity and yield of the target product ε-caprolactone. The evaluation results were illustrated in Table 3.

**Table 3**

| | Cyclohexanone conversion rate, % | ε-caprolactone selectivity, % | ε-caprolactone yield, % |
|---|---|---|---|
| Example 1 | 69.3 | 91.1 | 63.1 |
| Example 2 | 66.1 | 82.4 | 54.5 |
| Example 3 | 68.3 | 86.1 | 58.8 |
| Example 4 | 71.8 | 89.3 | 64.1 |
| Example 5 | 71.2 | 90.7 | 64.6 |
| Comparative Example 1 | 44.6 | 74.3 | 33.1 |
| Comparative Example 2 | 47.2 | 73.2 | 34.6 |

As indicated by the product properties of Table 1, Table 2 and the evaluation results of Table 3, the Sn-beta molecular sieves prepared by the present invention have a high Sn content, a high L acid content, and a high ratio of L acid content to B acid content, the properties allow the molecular sieves to show better activity and selectivity in the Baeyer-Villiger oxidation reaction of cyclic ketone.

## Claims

1. An Sn-Beta molecular sieve, is **characterized in that** the silicon-to-tin molar ratio of the molecular sieve is within the range of 8-15, and the molecular sieve has a L acid content within the range of 600-900 µmol/g as measured by pyridine infrared spectroscopy at a desorption temperature of 200°C.

2. The molecular sieve according to claim 1, wherein the silicon-to-tin molar ratio of the molecular sieve is within the range of 10-12, and the molecular sieve has a L acid content within the range of 700-800 µmol/g as measured by pyridine infrared spectroscopy at a desorption temperature of 200°C;
preferably, the molecular sieve has a ratio of L acid content to B acid content within the range of 70-120, more preferably within the range of 80-100 as measured by pyridine infrared spectroscopy at a desorption temperature of 200°C.

3. The molecular sieve according to claim 1, wherein the SiO₂/Al₂O₃ molar ratio of the molecular sieve is within the range of 2,700-4,000, preferably within the range of 3,000-3,500.

4. The molecular sieve according to any one of claims 1-3, wherein the molecular sieve has a nanoscale crystallite size, preferably, the molecular sieve has an average crystallite size within the range of 30-100 nm, more preferably within the range of 50-70 nm.

5. The molecular sieve according to any one of claims 1-4, wherein the molecular sieve has a specific surface area within the range of 530-630m²/g, preferably within the range of 550-600m²/g;
preferably, the molecular sieve has an external specific surface area within the range of 130-180m²/g, more preferably within the range of 145-165m²/g.

6. The molecular sieve according to any one of claims 1-5, wherein the molecular sieve has a pore volume within the range of 0.32-0.43mL/g, preferably within the range of 0.36-0.4mL/g.

7. A method for preparing Sn-Beta molecular sieve, is **characterized in that** the method comprises the following steps:
(1) Subjecting a HY molecular sieve and a tin source to a dealumination and tin supplementation reaction to obtain a Sn-Al-FAU molecular sieve;
(2) Performing a crystal transformation on the Sn-Al-FAU molecular sieve in the presence of Beta zeolite seeds to obtain a Sn-Al-Beta molecular sieve;
(3) Subjecting the Sn-Al-Beta molecular sieve to a dealumination process and then subjecting to a tin supplementation treatment.

8. The method according to claim 7, wherein the SiO₂/Al₂O₃ molar ratio of the HY molecular sieve in step (1) is within the range of 3.8-5, preferably within the range of 4.2-4.6;
preferably, the mass content of Na₂O in the HY molecular sieve is within the range of 0.5-1.5%, more preferably within the range of 0.8-1.2%.

9. The method according to claim 7 or 8, wherein the dealumination and tin supplementation reaction in step (1) comprises: reacting the HY molecular sieve with a gaseous phase tin source under the dealumination and tin supplementation reaction conditions;
preferably, the dealumination and tin supplementation reaction conditions comprises: a reaction temperature within the range of 400-600°C, more preferably within the range of 450-550°C, a time of 0.5-5 hours, more preferably 2-3 hours; a partial pressure of the gaseous phase tin source is within the range of 2.5kPa-7.5kPa, more preferably within the range of 4kPa-6kPa;
preferably, the tin source is an anhydrous tin source, more preferably at least one selected from the group consisting of tin tetrachloride, methyltin-trichloride, dimethyltin-dichloride and trimethyltin-chloride.

10. The method according to any one of claims 7-9, wherein step (2) comprises: mixing Beta zeolite seeds, a template agent, a silicon source, a Sn-Al-FAU molecular sieve and a solvent to obtain a mixed solution, then performing a crystallization reaction, and calcining the crystallized solid product;
preferably, the molar ratio of the materials in the mixed solution is SiO₂ : (0.03-0.09) Al₂O₃ : (0.03-0.08) SnO₂ : (0.006-0.021) Na₂O : (0.1-0.8) template agent : (3-8) H₂O, more preferably SiO₂ : (0.05-0.07) Al₂O₃ : (0.05-0.07) SnO₂ : (0.009-0.018) Na₂O : (0.4-0.6) template agent : (5-7) H₂O;
preferably, the Beta zeolite seeds calculated in terms of silica account for 3-10wt%, more preferably 6-8wt% of the total amount of silica in the mixed solution;
preferably, the SiO₂/Al₂O₃ molar ratio of the Beta zeolite seeds is within the range of 20-35, more preferably within the range of 25-30.

11. The method according to claim 10, wherein the crystallization conditions in step (2) comprise: a crystallization temperature within the range of 138-145°C, preferably within the range of 140-142°C; and a crystallization time of 5-15 days, preferably 8-12 days;
the calcination conditions in step (2) comprise: a calcination temperature within the range of 500-600°C and a calcination time of 4-8 hours.

12. The method according to any one of claims 7-11, wherein the dealumination in step (3) comprises: reacting the Sn-Al-Beta molecular sieve with an acid solution;
preferably, the acid is at least one selected from the group consisting of nitric acid, hydrochloric acid and sulfuric acid;
preferably, the concentration of said acid solution is within the range of 2.5-5mol/L, more preferably within the range of 3.5-4.5mol/L;
preferably, the solid-liquid mass ratio of the Sn-Al-Beta molecular sieve to the acid solution is 1:30-1:100, more preferably 1:50-1:70;
preferably, the conditions for reacting the Sn-Al-Beta molecular sieve with the acid solution comprise: a temperature within the range of 30-90°C, more preferably within the range of 50-70°C; a time of 1-6 hours, more preferably 3-4 hours; and the acid treatment is performed for 2-5 times, more preferably 3-4 times.

13. The method according to any one of claims 7-12, wherein the tin supplementation treatment in step (3) comprises: reacting the dealuminated molecular sieve with a gaseous phase tin source under the tin supplementation treatment conditions;
preferably, the tin supplementation treatment conditions comprise: a reaction temperature within the range of 400-550°C, more preferably within the range of 450-500°C, a time of 2-7 hours, more preferably 4-6 hours; a partial pressure of the gaseous phase tin source is within the range of lkPa-4kPa, more preferably within the range of 2kPa-3kPa;
Preferably, the tin source is an anhydrous tin source, more preferably at least one selected from the group consisting of tin tetrachloride, methyltin-trichloride, dimethyltin-dichloride and trimethyltin-chloride.

14. Use of the Sn-Beta molecular sieve according to any one of claims 1-6 in a cyclic ketone oxidation reaction, a saccharide isomerization reaction, a reaction for producing methyl lactate from glucose, a reaction for producing L-lactide from lactic acid, a propane dehydrogenation reaction, or a hydrogen transfer reaction between aldehyde ketones and alcohols, preferably the use in a cyclic ketone oxidation reaction, and more preferably the use in a cyclohexanone oxidation reaction.

15. An oxidation reaction method for a cyclic ketone, is **characterized in that** the method comprises: reacting a cyclic ketone with an oxidant under oxidation reaction conditions in the presence of a solvent and a catalyst;
the catalyst is the Sn-Beta molecular sieve according to any one of claims 1-6;
preferably, the cyclic ketone is at least one selected from the group consisting of cyclohexanone, 2-adamantanone, dihydrocarvone and bicyclo[3.2.0]hept-2-en-6-one, more preferably cyclohexanone;
preferably, the oxidant is at least one selected from the group consisting of tert-butyl hydroperoxide, hydrogen peroxide, cumyl hydroperoxide, cyclohexyl hydroperoxide, peracetic acid and peroxypropionic acid, more preferably hydrogen peroxide;
preferably, the used amount of the cyclic ketone is 0.4-1.4 mmol relative to 1 mmol of the oxidant;
preferably, the used amount of the catalyst is 10-25 mg relative to 1 mmol of the oxidant;
preferably, the oxidation reaction conditions comprise: an oxidation reaction temperature within the range of 75-95°C, and an oxidation reaction time of 2-8 hours.
